Europäisches Patentamt

European Patent Office

Office européen des brevets

(19)

(11) Publication number: 0 249 477
A2

(12) EUROPEAN PATENT APPLICATION

(21) Application number: 87305173.4

(22) Date of filing: 11.06.87

(51) Int. Cl.⁴: C 12 P 21/02
C 12 N 15/00

(30) Priority: 12.06.86 US 873497  12.06.86 US 873620

(43) Date of publication of application:
16.12.87 Bulletin 87/51

(84) Designated Contracting States:
AT BE CH DE ES FR GB GR IT LI LU NL SE

(71) Applicant: IMMUNEX CORPORATION
51 University Street Suite 600 600 Immunex Building
Seattle Washington 98101 (US)

(72) Inventor: Cerretti, Douglas Pat
1607 North 197th Place
Seattle Washington 98133 (US)

Clevenger, William Russell
7740 12th Avenue Northeast
Seattle Washington 98117 (US)

Cosman, David John
116th 11th Avenue East, No. 501
Seattle Washington 98102 (US)

Gimpel, Steven Dean
7727 44th Avenue Northeast
Seattle Washington 98115 (US)

Price, Virginia Lee
2617 Boyer Avenue East
Seattle Washington 98102 (US)

Urdal, David Lloyd
6826 55th Avenue Northeast
Seattle Washington 98115 (US)

(74) Representative: Sheard, Andrew Gregory et al
Kilburn & Strode 30, John Street
London WC1N 2DD (GB)

(54) Functional recombinant analog polypeptides devoid of hydrophobic amino acids.

(57) Functional recombinant analog proteins corresponding to native proteins are disclosed, having amino acid substitutions or deletions which eliminate residues constituting a hydrophobic transmembrane region. The corresponding native proteins may be lymphokine or lymphokine receptor or human CSF-1. Particular analog polypeptides have an amino acid sequence corresponding to amino acids 1-166 or 1-158 of Figure 1.

```
CAG CAG GTG TCG GAG TAC TGT AGC CAC ATG ATT CCG AGT CCA CAC CTG CAC TCT CTC CAG        60
Glu Gln Val Ser Glu Tyr Cys Ser His Met Ile Gly Ser Gly His Leu Gln Ser Leu Gln        20

CGC CTC ATT GAC AGT CAG ATC GAG ACC TCG TCC CAA ATT ACA TTT GAG TTT GTA CAC CAG       120
Arg Leu Ile Asp Ser Gln Met Glu Thr Ser Cys Gln Ile Thr Phe Glu Phe Val Asp Gln        40

GAA CAG TTG AAA GAT CCA GTC TGC TAC CTT AAC AAG GCA TTT CTC CTC CTA CAA TAC ATA       180
Glu Gln Leu Lys Asp Pro Val Cys Tyr Leu Lys Lys Ala Phe Leu Leu Val Gln Tyr Ile        60

ATG GAG GAC ACC ATG CGC TTC AGA GAT AAC ACC CGC AAT GCC ATC CCC ATT CTC CAC CTC       240
Met Glu Asp Thr Met Arg Phe Arg Asp Asn Thr Arg Asn Ala Ile Pro Ile Leu His Leu        80

CAG CAA CTC TCT TTG AGG CTG AAG ACC TGC TTC ACC AAG GAT TAT GAA CAG CAT CAC AAG       300
Gln Gln Leu Ser Leu Arg Leu Lys Ser Cys Phe Thr Lys Asp Tyr Glu Gln His His Lys       100

GCC TGC GTC CGA ACT TTC TAT GAG ACA CCT GTC CAG TTG CTG GAG AAG GTC AAG AAT GTC       360
Ala Cys Val Arg Thr Phe Tyr Glu Thr Pro Leu Gln Leu Leu Glu Lys Val Lys Asn Val       120

TTT AAT GAA ACA AAC AAT CTC CTT CAC AAG CAC TGG AAT ATT TTC AGC AAG AAC TGC AAG       420
Phe Asn Glu Thr Asn Asn Leu Leu Asp Lys Asp Trp Asn Ile Phe Ser Lys Asn Cys Asn       140

AAC AGC TTT GCT GAA TGC TCC AGC CAA GGG CAT CAC AGG CAG TCC GAG GGA TCC TCC AGC       480
Asn Ser Phe Ala Glu Cys Ser Ser Gln Gly His His Arg Gln Ser Glu Gly Ser Ser Ser       160

CCG CAC CTC CAG GAG TCT GTC TTC CAC CTG CTG GTG CCC ACT GTC ATC CTG GTC TTG CTC       540
Pro His Leu Gln Glu Ser Val Phe His Leu Leu Val Pro Ser Val Ile Leu Val Leu Leu       180

GCC GTC GGA GGC CTC TTG TTC TAC AGG TGG AGG CGC CGG AGC CAT CAA GAG CCT CAG AGA       600
Ala Val Gly Gly Leu Leu Phe Tyr Arg Trp Arg Arg Arg Ser His Gln Glu Pro Gln Arg       200

GCG GAT TCT CCG TTG GAG CAA CCA GAG GGG AGC CCC CTG ACT CAC GAT GAC AGA CAG GTC       660
Ala Asp Ser Pro Leu Glu Gln Pro Glu Gly Gly Ser Pro Leu Thr Gln Asp Asp Arg Gln Val       220

GAA CTG CCA GTC                                                                        672
Glu Leu Pro Val                                                                        224
```

Fig.1.

EP 0 249 477 A2

**Description**

FUNCTIONAL RECOMBINANT ANALOG POLYPEPTIDES DEVOID OF HYDROPHOBIC AMINO ACIDS

The present invention relates to recombinant DNA technology and, more particularly, to the cloning and expression of recombinant functional analog proteins and polypeptides (hereinafter collectively "proteins") not easily expressed in biologically active states due to the presence of a hydrophobic membrane spanning region.

Recombinant DNA technology has made it possible to produce relatively large quantities of homogeneous protein products once the gene encoding the desired protein has been isolated and sequenced. In one common procedure, the gene encoding a desired protein product is isolated from a cDNA library constructed by reverse transcription of polyadenylated mRNA isolated from total RNA extracted from cell lines or other potential sources of the desired protein product. The cDNA is inserted into an appropriate expression vector which is used to transform a host for the expected expression of the desired protein product. Such hosts typically include bacteria, such as Escherichia coli, yeast or other unicellular microorganisms. It has been found that some recombinant proteins are not expressed or are expressed at very low levels by host microorganisms or that the expressed protein products do not exhibit biological activity. There may be numerous reasons for this, including: ineffective promoters; degradation of the protein, for instance, by a protease encoded by the host cell; or, improper of incomplete posttranslational processing of precursor protein products. Also, the expressed protein may be biologically inactive if the correct tertiary structure of the protein is not attained, for instance, due to the absence of needed or the presence of undesired inter- or intramolecular disulfide bonds.

In addition, applicants have discovered that protein products having a region of highly hydrophobic amino acids near the carboxyl-terminus of the protein (a transmembrane region) may not be expressed in biologically active form by certain hosts or may only be expressed in limited levels. The presence of highly hydrophobic regions, especially at or near the carboxyl-terminus of the protein, may cause the expressed protein to be retained in the membrane of the host cell rather than being secreted. Such hydrophobic regions may, when present in multiple molecules, cause severe aggregation. Thus, if the nucleic acids encoding for such transmembrane regions are removed from the cDNA corresponding to the desired protein, perhaps expression of the protein would not be restricted. However, a potential problem that might be caused thereby is the deletion of amino acids that are required for biological activity.

The present invention may be used in conjunction with any protein normally having a membrane spanning region. Such proteins would include certain lymphokines such as human macrophage-specific colony stimulating factor, and all hormone receptors including lympyhokine receptors such as receptors to interleukin 1, interleukin 2 and granulocyte-macrophage colony stimulating factor.

In its most preferred aspect, the present invention concerns producing biologically active CSF-1 by altering the cDNA to eliminate nucleotides that encode highly hydrophobic amino acids in the carboxyl-terminal region of the protein molecule, or by synthesizing DNA fragments that are devoid of nucleotides found in the 3' region of the cDNA that encode this carboxyl-terminal region. CSF-1 is one member of a family of lymphokines known as colony stimulating factors (CSF's) that include hematopoietic stem cells to proliferate into large numbers of progenitor cells and then cause differentiation of such cells to form mature lymphocytes, mature neutrophilic and eosinophilic granulocytes, mature macrophages, mature megakaryocytes and monocytes.

CSF-1 (also known as mCSF) is a CSF that stimulates proliferation of progenitors into mature macrophages. Macrophages are relatively large (10-20 μm), actively motile phagocytic cells that develop from blood-borne monocytes which are hematopoietic in origin. When activated, they destroy foreign particles and decrepit cells, and also may provide resistance to or eradication of neoplastic disease.

Investigation of the biology and biochemistry of CSF-1 and its possible use in treatment of infection and neoplastic disease has been hampered, at least in part, by the unavailability of sufficiently large quantities of homogeneous CSF-1 to enable it to be thoroughly studied. Efforts to produce CSF-1 by recombinant techniques have met with only limited success. Heretofore it has not been possible to express biologically active human CSF-1 in bacterial or yeast expression systems. Although recombinant CSF-1 has been expressed by eukaryotic cells (monkey COS-7 cells), such expression is transient in nature with gene expression being lost after two or three divisions of the cells.

The present invention provides biologically active protein analogs such as recombinant analog CSF-1, encoded by mutant structural genes that differ from the cDNA for the native protein by the absence of nucleic acids that encode highly hydrophobic amino acids in the 3' terminal portion of the cDNA. Mutant genes encoding biologically active analog CSF-1 may be prepared by chemical synthesis, by mutation of the cDNA for the protein or by other appropriate method. The term "mutant gene" refers to a gene that differs from the cDNA of the native protein by absence, deletion or substitution of one or more nucleic acids or codons. The gene may be chemically synthesized, cloned from cDNA, mutated from genomic DNA or otherwise prepared. The term "protein analog" refers to proteins having essentially the same biological activity as the corresponding native protein but differing in amino acid composition from the protein product encoded by the native cDNA. "Mutant CSF-1" refers to a mutant gene encoding a protein analog of CSF-1 having essentially the same activity as native CSF-1 but differing in composition from CSF-1 encoded by native CSF-1 cDNA ("CSF-1 analog").

The present invention also provides truncated structural mutant CSF-1 genes characterised by deletion of nucleotides located between the nucleotides encoding the highly hydrophobic amino acids of the transmembrane region and the 3' terminal of the native cDNA.

Subaspects of the present invention include cloning and expression vectors which contain the aforementioned mutant genes, and use of such vectors to transform host cells for expression of biologically active analog CSF-1.

A further aspect of the present invention concerns therapeutic compositions of the recombinant, analog biologically active CSF-1 which are useful in the treatment of infection and neoplastic disease. A related aspect of the present invention concerns therapeutic methods that employ the therapeutic compositions of the present invention.

Another aspect of the present invention concerns a rapid biological assay for testing for the presence of functional CSF-1. In the assay, the ability of CSF-1 to induce proliferation of murine mononuclear phagocytes in the presence of tritiated thymidine is ascertained.

Various embodiments of the invention will now be described, with reference to the drawings, in which:

FIGURE 1 illustrates the nucleic acid sequence and the deduced amino acid sequence of the cDNA for human CSF-1;

FIGURE 2 depicts the nucleic acid sequence and the corrosponding amino acid sequence for a hujman CSF-1 gene of the present invention, with the N-terminus of the mature protein being marked with an arrow, with restriction enzyme cleavage sites indicated and with nucleotide changes from the cDNA illustrated in FIGURE 1 being indicated in lower case letters;

FIGURE 3 depicts the composition of chemically synthesized oligonucleotides employed to form the mutant CSF-1 gene depicted in FIGURE 2;

FIGURE 4 illustrates the strategy employed to construct the p FCSF-1 plasmid with the CSF-1 mutant gene depicted in FIGURE 2 inserted therein, for use in transforming E. coli cells to replicate the plasmid and yeast host cells to express biologically active analog CSF-1; and

FIGURE 5 illustrates the strategy employed to construct a shuttle vector with a mutant CSF-1 gene derived from in vitro mutagenesis of the CSF-1 cDNA depicted in FIGURE 1 inserted therein for use in transforming E. coli cells to replicate the plasmid and yeast host cells to express biologically active CSF-1.

The present invention relates to high level expression of functional, analog proteins such as CSF-1 by recombinant DNA techniques involving deletion of highly hydrophobic amino acids encoded by the cDNA, located in the carboxyl terminal region of the protein and which are not essential to the biological activity of the expressed protein. The presence of such amino acids may inhibit the expression of mature, functional protein by unicellular hosts due to the post translation protein product being bound to the membrane of the host cell rather than being secreted by the host or due to hydrophobic interactions leading to complex associations of the protein molecules which in turn alter the conformation of individual molecules such that biological activity is lost. Deletion of hydrophobic amino acids from the carboxyl-terminal region may however affect the biological activity of the protein or even render it inactive, for example, by altering the tertiary structure of the protein. Thus, determining what particular amino acids are to be deleted is not necessarily a straightforward procedure.

Once the desired composition of the CSF-1 or other analog has been determined, a corresponding mutant gene encoding the analog is prepared. The cDNA corresponding to the analog may be isolated from a cDNA library, cloned in an appropriate cloning system and then sequenced by known techniques. The cDNA is then altered to delete selected nucleic acids that encode undesired hydrophobic amino acids. In an alternative procedure, the mutant structural gene may be chemically synthesized so that the resultant DNA fragment is formed without the nucleic acids encoding the undesired carboxyl-terminal hydrophobic amino acids.

Although the present invention is specifically described and exemplified below with respect to the lymphokine CSF-1, the present invention is applicable to any protein having a segment of highly hydrophobic amino acids, especially in the carboxyl-terminal portion of the protein, that prevents or restricts the expression of the protein in biologically active form. Examples of proteins other than CSF-1 with respect to which the present invention may be employed include other lymphokines and hormone receptors, including lymphokine receptors, for example, receptors to interleukin 1, interleukin 2 and granulocyte-macrophage colony stimulating factor. The present invention may also be used in conjunction with oncogenes, such as myc, ras, fos, fes, fms, sarc, erb, abl, etc.

Synthesis of Mutant Structural Gene

DNA encoding a desired composition of the CSF-1 analog, with the sequences encoding undesired hydrophobic amino acids deleted, may be chemically synthesized by well-known techniques, such as by phosphodiester or triester methods. The details of triester synthesis are set forth in Sood et al., Nucl. Acids Res. 4:2557 (1977); and, Hirose et al., Tet. Lett. 28:2449 (1978). An advantage of synthesizing the mutant CSF-1 gene is that the gene can be produced with desired leader or promoter sequences to enhance high level expression of the CSF-1 analog in a chosen host. Also, the CSF-1 gene can be designed with cohesive termini to facilitate insertion of the CSF-1 analog in a chosen vector. Further, nucleotides can be altered from the wild-type CSF-1 gene to provide unique restriction enzyme cleavage sites without changing the amino acids that are encoded by the resulting codons. Such restriction enzyme cleavage sites would be of assistance in gene selection procedures used during the cloning process, discussed below. In addition, synthesis of the mutant CSF-1 gene enables introns existing in genomic DNA to be eliminated as well as codons which encode amino acid residues which are not essential to the biological activity of the resulting protein product. Another advantage of chemically synthesizing the mutant CSF-1 gene is to eliminate amino acid sequences which are cleaved by enzymes and proteases encoded by genes native to the host cell. For instance, in published European Patent Application 0212914, applicants and co-researchers discuss elimination of multi-basic amino acids to prevent post translation cleavage of proteins by the protease encoded by the KEX-2 gene of the yeast Saccharomyces cerevisiae.

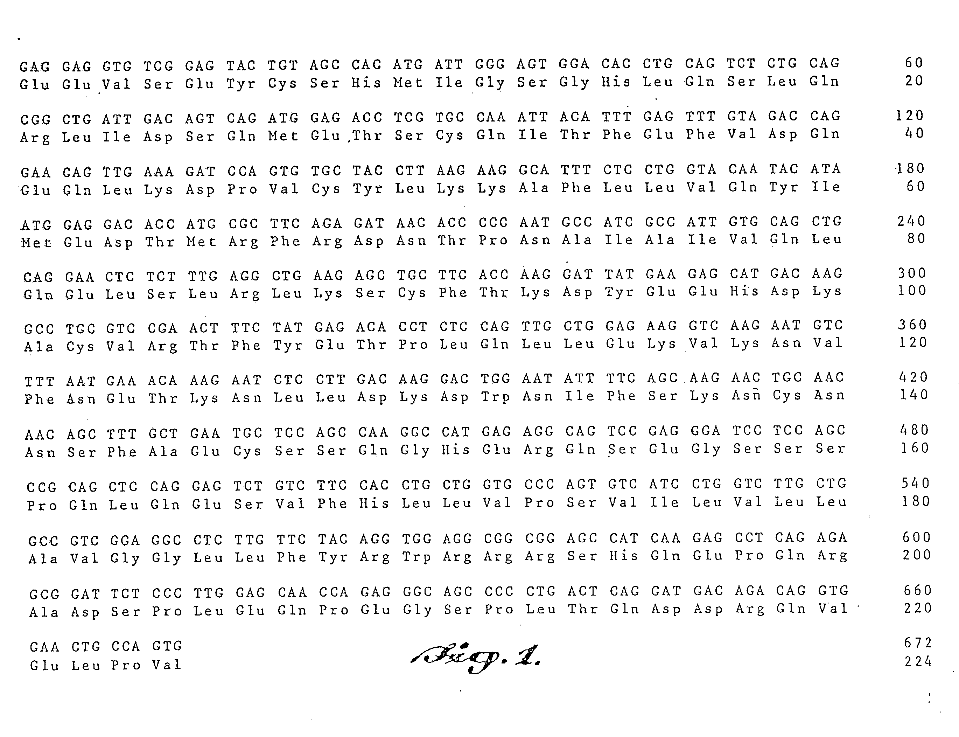

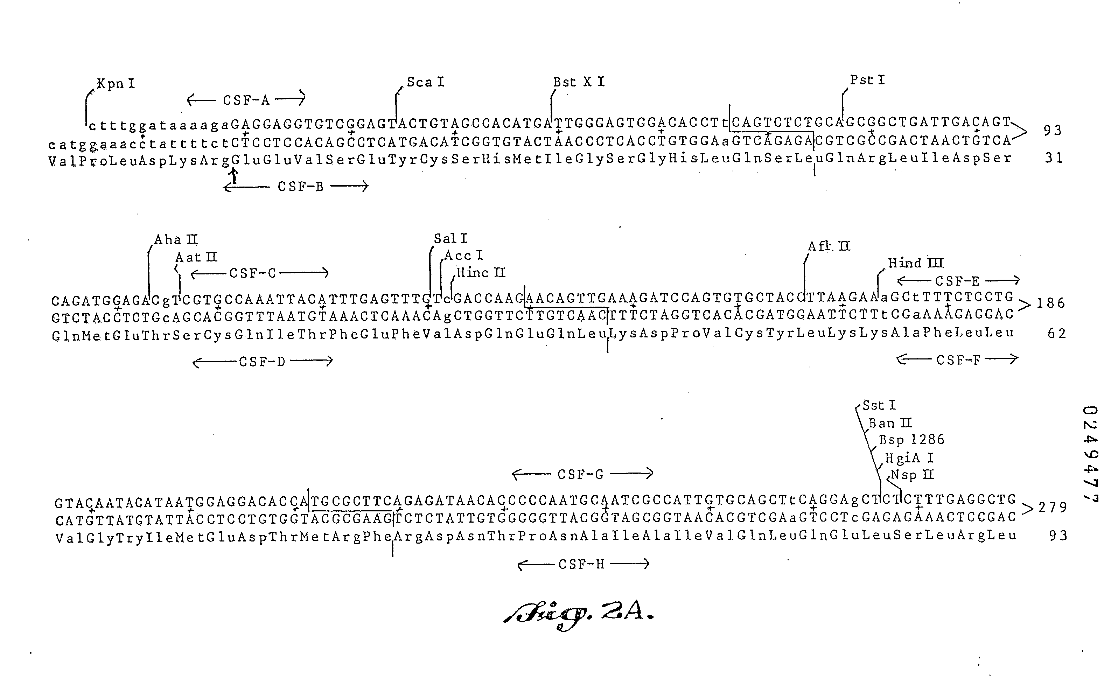

The nucleic acid sequence of the cDNA encoding human CSF-1 is set forth in FIGURE 1. See Kawasaki et al., Science 230:291 (1985). The cDNA clone in FIGURE 1 is isolated from a cDNA library constructed by reverse transcription of polyadenylated mRNA derived from a pancreatic human cell line, MIA-PaCA-2. The deduced amino acid sequence of the CSF-1 cDNA is set forth below corresponding codons. As apparent from FIGURE 1, a segment of the carboxyl-terminal of the encoded protein is composed of mostly hydrophobic amino acids, e.g., amino acid residue Nos. 166 through 188. Heretofore it has only been possible to express biologically active CSF-1 with the cDNA in FIGURE 1 in monkey COS cells. Attempts to express this protein in bacterial and yeast cells have been unsuccessful. The COS expression system is not of practical value in that it is transient in nature; gene expression is lost after two or three divisions of the COS cells.

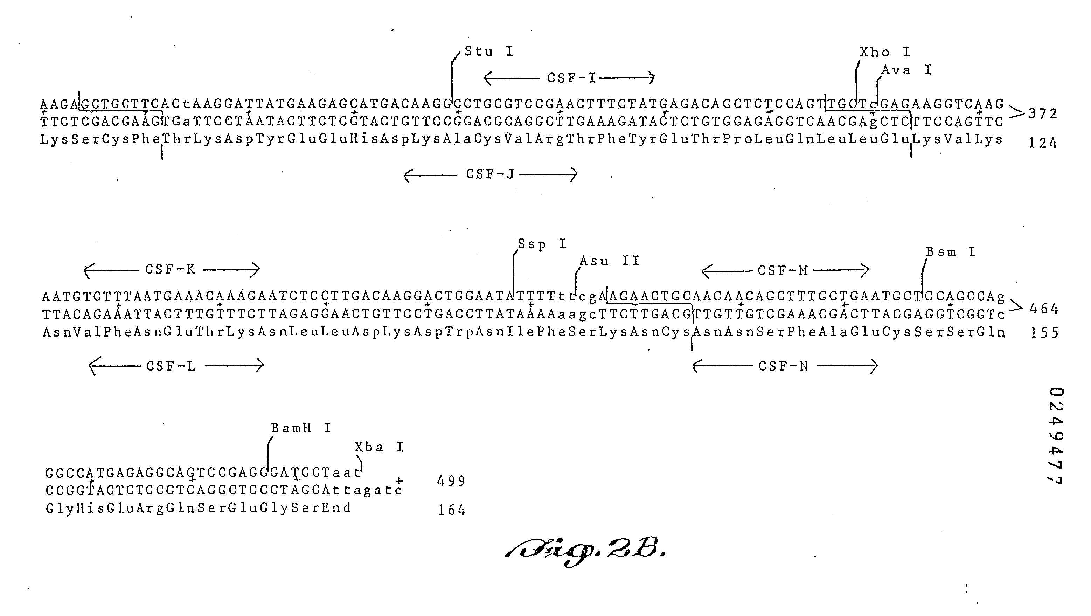

In accordance with the present invention, a mutant CSF-1 gene is chemically synthesized without the nucleotides encoding the carboxyl-terminal hydrophobic amino acids. To this end, the mutated gene preferably is synthesized with at least the nucleic acids encoding amino acids 166-188 in FIGURE 1 (nucleic acid Nos. 496 through 564) deleted. Preferably the mutated CSF-1 gene is synthesized as a truncated form of the CSF-1 cDNA in FIGURE 1, by also deleting the nucleotides located 3' of the deleted nucleotides that encode the hydrophobic amino acids. Ideally, the mutated CSF-1 gene is synthesized with one or more nucleic acids located adjacent the 5' end of the section of nucleotides encoding the highly hydrophobic amino acids also deleted. This has the twofold purpose of helping to ensure that the transmembrane portion of the CSF-1 is not encoded and to shorten the protein product thereby facilitating its expression while not significantly altering the activity of the protein. Thus, the mutant CSF-1 gene may be truncated so that its 3' end terminates at nucleic acid No. 474 (amino acid No. 158 in FIGURE 1). A mutant CSF-1 gene of such composition is shown in FIGURE 2.

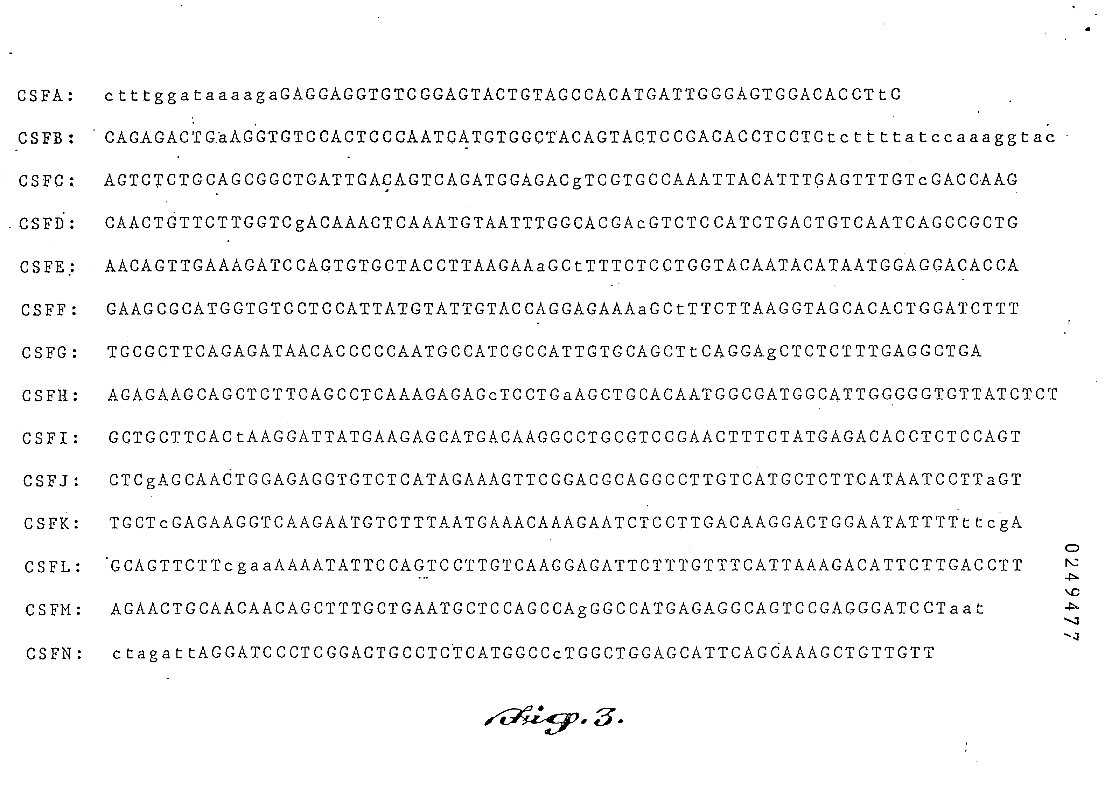

The CSF-1 gene shown in FIGURE 2 may be prepared by chemically synthesizing a series of oligonucleotides which are then assembled in duplex form to constitute the gene. To aid in their proper enzymatic ligation, the oligonucleotides are prepared with cohesive termini or "sticky-ends." Also, the enable the oligonucleotides to be ligated together, their 5' ends are phosphorylated with the deoxyribonucleotide triphosphate dATP in the presence of $T_4$ polynucleotide kinase. Thereafter, the oligonucleotides are ligated with $T_4$ ligase and dATP. The resulting assembled DNA fragments that have the desired composition, as set forth in FIGURE 3, are selected from the ligation reaction by electrophoresis. The sequence of the selected mutant CSF-1 gene is confirmed by standard restriction enzyme analysis or by DNA sequencing using the procedures set forth in Example A below.

A mutant CSF-1 gene may be composed of the 14 oligonucleotides depicted in FIGURE 3. The relative location of these oligonucleotides along the mutated CSF-1 gene are shown in FIGURE 2. The 14 complementary oligonucleotides are synthesized with cohesive termini, each 8 base pair ("bp") in length, so that they can be readily ligated in proper order and orientation. These cohesive termini do not correspond to the cleavage sites of any known restriction enzyme. Also, certain nucleotides are changed from the wild-type sequence of the CSF-1 gene shown in FIGURE 1. These alterations are shown in lower case letters in FIGURES 2 and 3. By this procedure, the mutant CSF-1 gene is constructed with 24 unique restriction enzyme cleavage sites, including Kpn I and Xba I sites at the 5' and 3' termini, respectively, of the mutant gene. As shown in FIGURE 2, oligonucleotides CSF-A and CSF-B are synthesized with nucleic acids encoding six amino acids at the amino-terminal of the analog CSF-1 of the following composition: Val-Pro-Leu-Asp-Lys-Arg. These six amino acids constitute the carboxyl-terminal end portion of a yeast pre-pro-α leader sequence which, as discussed below, facilitates high level expression of analog CSF-1 in a yeast system.

Once the desired mutant gene has been chemically synthesized, it is inserted within a vector which is used to transform compatible prokaryotic or eukaryotic host cells for replication of the mutant gene and expression of active protein product encoded by the mutant gene. In the present invention various cloning vectors may be utilized. Numerous plasmids especially adapted for replication of genes and/or high level expression of proteins encoded by the genes are widely commercially available. The particular plasmid chosen should be compatible with the contemplated transformation host, whether a bacteria such as E. coli, yeast, or other unicellular microorganism. The plasmid should include a proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as an antibiotic. Plasmids are commercially available that encode genes resistant to various antibiotics, including tetracycline, streptomycin, sulfa drugs, penicillin and ampicillin.

Transformation hosts for plasmid cloning/expression vectors may include any appropriate prokaryotic or eukaryotic cell; however, preferably it is a well-defined bacteria, such as E. coli, or a yeast strain. Such hosts are readily rendered competent and capable of rapid growth in culture.

In transformation protocols, typically a limited portion of the host cells are actually transformed. The cells

4

that have been transformed can be identified by placing the cell culture on agar plates containing a suitable growth medium and a phenotypic identifier, such as an antibiotic. Only those cells that have the proper resistance gene, e.g., to the antiobiotic, will survive. Such identified hosts are cultured, typically in large scale, for production of the desired protein product.

Large fragments of chemically synthesized oligonucleotides often cannot be successfully cloned. Although the reason for this is not entirely understood, it may result from "mistakes" occurring during the chemical synthesis of the oligonucleotide whereby an improper nucleic acid is synthesized. If this occurs to any significant extent, transcription or translation may not take place. With respect to the particular composition of the mutant CSF-1 gene set forth in FIGURE 2, applicants found that when this entire truncated gene was assembled directly from the 14 oligonucleotides shown in FIGURE 3 in a single ligation, and the subsequent approximately 500 bp fragment ligated into a cloning plasmid, transformation of E. coli with the plasmid was unsuccessful. The synthetic DNA as a large fragment was not replicated effectively in E. coli. As a result, an alternative cloning strategy was employed whereby smaller fragments of the synthetic DNA are first subcloned and then the subcloned segments assembled into a complete mutant gene.

In the alternative cloning strategy, the oligonucleotides in FIGURE 3 are assembled into two duplexed preliminary fragments each containing 280 bp. The first fragment, designated as Fragment I, consists of nucleotides CSF-A through CSF-H, and the second fragment designated as Fragment II, is composed of nucleotides CSF-G through CSF-N. As discussed above, the 5' ends of the oligonucleotides are phosphorylated in the presence of dATP and $T_4$ polynucleotide kinase to enable the oligonucleotides to be ligated together. The CSF-A and CSF-N oligonucleotides are not, however, phosphorylated thereby to prevent their "head-to-head" or "tail-to-tail" ligation. Ligation of the oligonucleotides to form Fragments I and II are carried out in reaction mixtures containing, inter alia, $T_4$ ligase and dATP. The two assembled preliminary DNA fragments are resolved from the ligation mixture by electrophoresis. Bands migrating at the expected sites of the preliminary fragments are sliced from the electrophoresis gel and purified by standard nucleic acid purification techniques, such as by ethanol precipitation and/or ion exchange chromatography.

Thereafter, Fragments I and II are digested with restriction enzymes to form three separate "final" fragments each having unique cohesive termini to facilitate their subsequent assembly into a complete mutant gene. In this regard, preliminary Fragment I is digested with the restriction enzymes Hind III and Sst I thereby to divide this fragment into two separate final fragments, a first fragment, designated as F-1, extending from nucleotides Nos. 1 through 174 and a second fragment, designated at F-2, extending from nucleotides Nos. 175-269 in FIGURE 2. Preliminary Fragment II is digested with the restriction enzyme Sst I thereby to form a singular fragment, designated as F-3, extending from nucleotides Nos. 270 through 500 in FIGURE 2. The three individual fragments are then inserted into cloning vectors and transformed into hosts for replication of the plasmids. Transformed hosts are selected with a phenotypic marker and then the replicated plasmids extracted from the transformants using any one of several well-known techniques, such as by alkaline lysis. The sequences of the three subclones are confirmed by DNA sequencing, for instance, by standard chain termination methods as originated by Sanger et al., Proc. Natl. Acad. Sci. USA 70:5463 (1977) and as detailed in Example A below. Thereafter, the colonies containing the three subclones are cultured for large scale plasmid preparation and then the replicated clones removed therefrom using standard techniques.

Preparation of Mutant Structural Genes from cDNA

Total human RNA is extracted from cell lines or other sources thought to produce relatively high levels of CSF-1, for instance, human lung cells or, as noted above, the pancreatic human cell line MIA-PaCa-2. See Das et al., Blood 58:630 (1981); Wu et al., Biol. Chem. 254:6226 (1979); and, Kawasaki et al., supra. Polyadenylated mRNA is isolated from the total RNA extract and then a cDNA library is constructed by reverse transcription of the polyadenylated mRNA with the enzyme reverse transcriptase. The DNA is rendered double-stranded with DNA polymerase I and inserted into an appropriate cloning vector. The resultant recombinant cloning vectors are then used to transform an appropriate host. Details of the above procedures are set forth in Maniatis et al., supra beginning at page 187.

Transformed hosts are identified and grouped in the pools. Plasmid DNA, which is prepared from these pools, is hybridized with a radiolabeled probe exhibiting homology to the human CSF-1 gene. For example, the oligonucleotide probe may be chemically synthesized to correspond to a portion of the human CSF-1 genomic clone which has been partially characterized by Kawasaki et al., supra.

The pool(s) of clones that give a positive signal to the probe is (are) identified and then the putative pool subdivided and the hybridization screen repeated. A single transformant corresponding to the human CSF-1 gene is eventually identified. The plasmid DNA is prepared from this transformant and characterized by DNA sequencing, for instance, using the chain termination method of Sanger et al., supra.

Next, nucleotides encoding hydrophobic amino acids located at or near the carboxyl-terminal portion of the CSF-1 cDNA are removed. As discussed above relative to the procedure for chemically synthesizing the mutant CSF-1 gene, the nucleic acids located 3' to those encoding the hydrophobic amino acids also may be removed. In addition, nucleic acids located adjacent the 5' end of the section of the nucleotides encoding the hydrophobic amino acids also may be removed as long as biological activity is not lost thereby. The removal of these targeted nucleic acids from the cDNA may be accomplished by standard in vitro mutagenesis techniques, such as by digesting the plasmid cDNA with a restriction enzyme which is capable of cleaving the cDNA at the desired location. As an alternative, in vitro mutation of the plasmid cDNA can be carried out by intially digesting the plasmid with restriction enzyme(s) to linearize the DNA. Thereafter both strands of the resulting DNA fragment may be simultaneously degraded with Bal 31 exonuclease until the desired nucleic acids are removed from the 3' end portion of the fragment. This procedure is especially useful in constructing mutant genes that constitute a truncated form of the cDNA. As a further alternative, various mutagenesis techniques can be used to remove a section of nucleotides located in an intermediate section of the cDNA, including site-specific mutagenesis procedures. Details of site-specific mutagenesis procedures are set forth in Craik, Biotechniques, Jan. 1985, 12-19. Once the desired composition of the cDNA mutant is prepared, it is inserted into a cloning vector for use in transforming an appropriate host for the replication of the plasmid and the expression of biologically active analog CSF-1, as discussed below.

Expression of Analog CSF-1 in Yeast Hosts

Applicants have discovered that the mutant gene depicted in FIGURE 2 is capable of high level expression of biologically active analog CSF-1 in yeast hosts. The present invention is not specifically limited to the expression of analog CSF-1 in yeast but may include other types of expression systems as well.

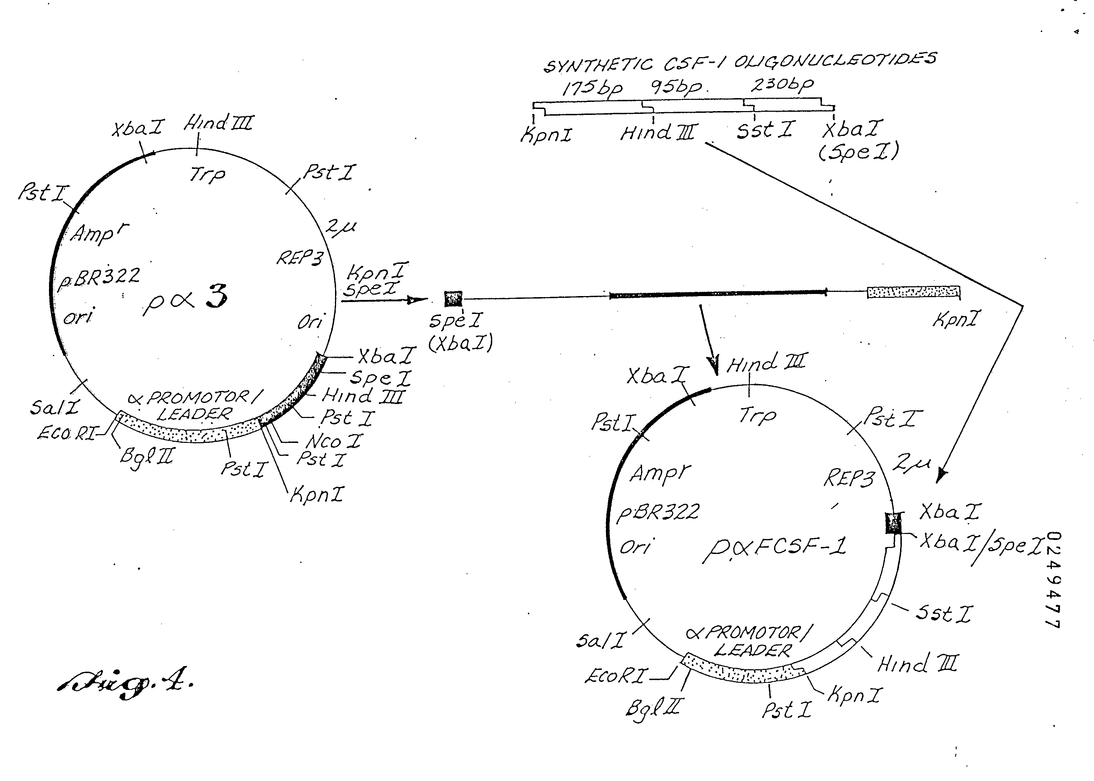

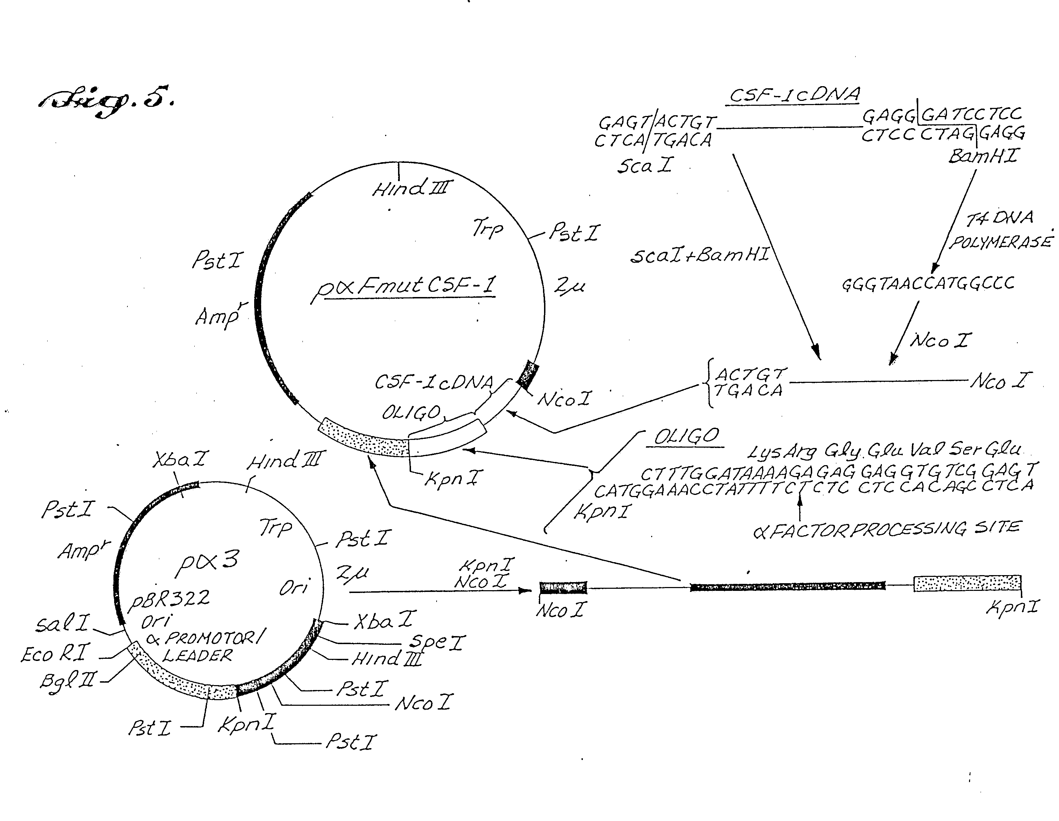

In the yeast expression system, the mutant CSF-1 gene shown in FIGURE 2 (composed of fragments F-1, F-2 and F-3 assembled as shown in FIGURE 4) is inserted into a shuttle vector designed for replication of the gene and subsequent expression of analog CSF-1 in bacteria and yeast host cells, respectively. As shown in FIGURE 4, the shuttle vector, designated as pαF CSF-1, preferably contains sequences derived from plasmid pBR332 including an origin of replication for E. coli and an ampicillin resistance gene (Ampr) as a selectable marker (thick line portion). Ideally, the expression vector also includes sequences from yeast, for instance, the tryptophan-1 gene (Trp-1) as a selectable marker and the 2 μ yeast origin of replication (thin line portion in FIGURE 4). Also ideally, the shuttle vector further includes the yeast α-factor promoter together with leader sequences to direct the high level synthesis and secretion of analog CSF-1 in yeast hosts (stippled box portion in FIGURE 4), followed by the sequences for the mutant CSF-1 gene (open box portion). The structure of the α-factor gene is discussed in Kurjan nd Herskowitz, Cell 30:933 (1982).

A ligation mixture consisting of the three DNA fragments composing the mutant CSF-1 gene (FIGURE 4), together with the digested shuttle vector is initially transformed into E. coli. Transformed E. coli hosts are selected and then the recombinant plasmids containing the desired construct are identified by standard restriction enzyme analysis. The unique restriction enzyme cleavage sites incorporated into the three synthetic DNA fragments F-1, F-2 and F-3 facilitate this procedure.

Shuttle vectors that contain the three DNA fragments in proper order are than transformed into an appropriate strain of S. cerevisiae. Preferable strains include, but are not limited to, yeast strains 79, X2181-1B, DBY746, YNN282, 20B-12. These strains are all α, Trp 1 for compatibility with the α-factor promoter and for selection of Trp+ transformants. These strains are all widely available, for instance strain 79 is available from the Yeast Genetic Stock Center, Department of BioPhysics and Medical Physics, University of California, Berkeley, CA 94702.

Transformation of the yeast host with the recombinant shuttle vector containing the mutant CSF-1 gene is conducted according to well-known procedures wherein spheroplasts are formed and then washed prior to plasmid uptake. Standard protocols for this procedure have been established. See Beggs, Nature (London) 275:104 (1978); and, Hinnen et al., Proc. Natl. Acad. Sci. USA 75:1929 (1978).

Assays for Biological Activity

The yeast cell culture supernatants were assayed for biological activity in standard colony forming assays (Example B below) and in a more rapid thymidine proliferation assay (Example C below). The colony forming assay ascertains the capacity of CSF-1 to induce proliferation and differentiation of bone marrow cells obtained from murine sources. In this assay, the resulting colonies are picked and then stained to visualize the types of cells (e.g., granulocytes, macrophages, neutrophils, eosinophils, etc) present in the colonies.

One drawback of the colony forming assay is the relatively long length of time required for its completion, i.e., 7 to 14 days. As an alternative to the traditional colony forming assay, the present invention provides a proliferation assay which is completed within 24 hours. The assay is based on the observations that human CSF-1 is biologically active on murine mononuclear phagocytes, whereas human GM-CSF does not exhibit this biological activity in the murine system. The assay tests for the presence of CSF-1 activity by ascertaining the capacity of samples putatively containing human CSF-1 to induce proliferation of murine mononuclear phagocytes. The details of this assay are set forth below in Example D.

On the basis of the colony forming assay and the thymidine proliferation assay, the yeast fermenation fluids were found to contain relatively high levels of CSF-1 activity. The levels measured were approximately 45,000 CSF-1 colony forming units per milliliter (CFU/ml), or approximately 1 microgram (μg) of recombinant analog CSF-1 per milliliter (ml) of fermentation fluid.

Therapeutic Applications

The analog CSF-1 of the present invention exhibits the biological activity of native CSF-1. As such, it can be utilized in therapeutic, diagnostic or research applications in the same manner as native CSF-1, for instance: for diagnosis and treatment of infection and neoplastic disease; for augmenting cell-mediated cytotoxicity; for stimulating lymphokine activated killer cell activity; for the development of diagnostic assays for monitoring CSF-1 levels in disease states; and, for the induction in vivo of leukocytosis and/or monocytosis. The analog

6

CSF-1 of the present invention may be used by itself or in combination with other immunologically effective B or T cell lymphokines or other therapeutic agents. Examples of useful therapeutic agents include macrophage activating factor (MAF) and the various species of interferons.

For therapeutic or diagnostic applications analog CSF-1 may be formulated in a suitable nontoxic, nonallergenic, physiologically compatible carrier, such as distilled water, saline, saline mixed with human serum albumin, Ringer's solution, Hank's solution, etc. The CSF-1 may be administered by any suitable method, including orally or by injection intraperitoneally, intramuscularly or subcutaneously. For therapeutic applications, doses of CSF-1 may be in the range of 0.01 to 10,000 µg per dose.

## EXAMPLE A

### Nucleic Acid Sequencing of DNA Fragments

DNA fragments, including the subcloned fragments F-1, F-2 and F-3 used to assemble the mutant CSF-1 gene, were sequenced by the standard chain-termination method as originated by Sanger et al., supra and described in U.S. Pat. No. 4,322,499, and detailed in the Amersham Handbook entitled M13 Cloning and Sequencing, Blenheim Cresent, London (1983) (hereinafter "Amersham Handbook"). See also Messing, 2 Recombinant DNA Technical Bulletin, NIH Publication No. 79-99, 2, 43-48 (1979); Norrander et al., Gene 26:101 (1983); Cerretti et al., Nucl. Acids Res. 11:2599 (1983); and, Biggin et al., Proc. Natl. Acad. Sci (USA) 80:3963 (1983).

In the sequencing procedure, M13 filamentous phage is employed as a vector to clone the DNA sequence of interest. These phage vectors provide single-stranded DNA templates which are readily sequenced by the chain-termination method. This method involves priming a single-stranded template molecule with a short primer strand having a free 3′ hydroxyl group and then using DNA polymerase (Klenow fragment) to copy the template strand in a chain extension reaction using all four deoxyribonucleotide riphosphates, i.e., dATP, dCTP, dGTP, and dTTP (collectively referred to as "dNTPs"), with one of the dNTP's being radiolabeled. In the synthesis reaction, a nucleotide specific chain terminator lacking a 3′ hydroxyl terminus, for instance, a 2′, 3′ dideoxynucleotide triphosphate ("ddNTP"), is used to produce a series of different length chain extensions. The terminator has a normal 5′ terminus so that it can be incorporated into a growing DNA chain, but lacks a 3′ hydroxyl terminus. Once the terminator has been integrated into a DNA chain, no further deoxynucleotide triphosphates can be added so that growth of the chain stops. Four separate synthesizing reactions are carried out, each having a ddNTP of one of the four nucleotide dNTP's, i.e., dATP, DCTP, dGTP and dTTP. One of the normal dNTP's is radiolabeled so that the synthesized strands, after having been sorted by size on a polyacrylamide gel, can be autoradiographed. The chain extensions from the four reactions are placed side by side in separate gel lanes so that the pattern of the fragments from the autoradiography corresponds to the nucleic acid sequence of the cloned DNA.

The DNA fragments of the present invention were sequenced essentially as described in the Amersham Handbook, supra, with the variations set forth below. The fragments were digested with Pst I and/or Rsa I and then subcloned into strains mp18 and mp19 of the M13 single-stranded filamentous phage vector (Amersham, Arlington Heights, IL). The mp18 and mp19 phage vectors, as set forth in Norrander et al., supra, contain the following unique cloning sites: Hind III; Sph I; Pst I; Sal I; Acc I; Hinc II; Xba I; BamHI; Xma I; Smal; Kpn I; Sst I; and, Eco RI. The composition of the mp18 and mp19 vectors are identical, with the exception that the order of the above-identified restriction sites are reversed in the mp19 vector so that both strands of the cDNA insert may be conveniently sequenced with the two vectors. The mp18 amd mp19 vectors, with a corresponding strand of the cDNA inserted therein, were used to transform E. coli JM107 of the strain K12 (Bethesda Research Laboratories, Bethesda, MD) to produce replicate single-stranded DNA templates containing single-stranded inserts of the sense and antisense strands.

The synthetic universal primer: 5′-CCCAGTCACGACGTT-3′ (P-L Biochemicals, Milwaukee, WI), was annealed to the single-strand DNA templates and used to prime DNA synthesis as described above. Thereafter, the extension fragments were size-separated by gel electrophoresis and autoradiographed from which the nucleotide sequences of the fragments were deduced.

Deoxyadenosine 5′(α-[$^{35}$S] thio) triphosphate (hereinafter "dATP [α-$^{35}$S]") was used as the radioactive label in the dideoxy sequencing reactions. Also, rather than using the gel set forth at page 36 of the Amersham Handbook, a 6% polyacrylamide gel was employed (6% polyacrylamide gel, 0.4 mm thick, containing 7 M urea, 100 mM Tris borate [pH 8.1], and 2 mM ethylenediaminetetraacetate ("EDTA").

## EXAMPLE B

### Murine Bone Marrow Colony Assay

This assay ascertained the capacity of the analog CSF-1 of the present invention to stimulate the formation of differentiated macrophage colonies in semi-solid cultures of bone marrow stem cells. The cells from the colonies that were generated were stained with Orcein to visualize the cells that colonized.

The assay employed a stock solution of nutrient medium and stock agar composition. The agar was composed of 1.4 bacto-agar (Difco, Detroit, MI) in sterile distilled water. The stock nutrient medium, which may be stored for up to two weeks at 4°C, was of the following composition:

1. 28.5% horse serum;

2. $0.7 \times 10^4$ M 2-mercaptoethanol;

3. 0.12 mg/ml asparagine;

4. 0.7 mg/ml glutamine;

5. 150 U/ml of penicillin G;

6. 150 U/ml of streptomycin;

7. $1.1 \times \alpha$-minimum essential medium ($\alpha$MEM); and,

8. $2.2 \times$ vitamins (Gibco Laboratories, Chagrin Falls, OH), Cat #320-1120).

In the assay procedure, 50 microliters ("$\mu$l") samples were plated in appropriate wells in a log-2 dilution series. The agar for the bacto-agar solution, identified above, was prepared by placing the tube containing the agar suspension in a boiling water bath for approximately 10 minutes. Once the agar was in solution, it was transferred to a 40°C bath.

The nutrient medium was warmed to 37°C, and then seven parts of the medium were mixed with three parts of the bacto-agar solution (hereinafter "incubation medium") and maintained at 37°C. Percoll treated bone marrow cells were then warmed to 37°C and immediately added to the incubation medium at a final concentration of approximately $5 \times 10^4$ cells/ml. The bone marrow cell mixture was kept at 37°C while dispensing 250 $\mu$g aliquots into each well of the plate containing the samples to be tested. The plates were then swirled gently and allowed to sit at room temperature until the agar hardened. Thereafter the plates were placed in plastic boxes containing distilled water to prevent the wells from drying out.

Colonies having 50 or more cells each were counted on days 4,5 and 7; the earlier time being better for granulocyte colonies, and the latter time being better for macrophage and mixed colonies. In each assay, several wells were plated without CSF-1 samples to obtain a background colony count. The average number of colonies that grew in the blank wells were subtracted from the number of colonies found in each of the wells containing the samples. The activity in CFU/ml of the analog CSF-1 in the test samples was determined as being equal to the dilution of the sample at which the colony number is one-half of the maximum colonies formed by $1 \times 10^5$ bone marrow cells multiplied by the number of colonies observed in the half maximal case. In other words if a sample generated half maximal colonies (i.e., 35) at a dilution of 1:1000, that sample was said to contain 35 x 1000 or 35,000 CRU/ml.

The types of cells in the colonies were determined by picking the colonies and staining individual cells with a stain composed of 0.6% Orcein and 60% acetic acid. In the staining procedure, an equal volume of 50% methanol (MeOH) was added to each of the cultures and then the cultures were incubated at room temperature for 20 minutes. Thereafter, the 50% MeOH was aspirated off and then an equal volume of 100% MeOH was added to each culture, followed by incubation at room temperature for 20 minutes or overnight at 4°C. Next, the 100% MeOH was aspirated off and culture dried.

The Orcein stain mixture was added to the cell culture in an amount equal to 50% of the original volume of the culture. After approximately 20 minutes, the nuclei of the cells were visible. Thereafter, cultures were flooded with distilled water and then aspirated to remove the Orcein (which had precipitated). Next, distilled water in a volume equal to the original volume of a culture, was added which intensified the color of the stain and made it easier to evaluate the cell composition of the colonies.

EXAMPLE C

Thymidine Proliferation Assay

As noted above, this assay ascertains the capacity of the analog CSF-1 of the present invention to induce proliferation of murine mononuclear phagocytes derived from non-adherent bone marrow cells. Tushinski et al., Cell 28:71 (1982); Tushinski and Stanley, J. Cell. Physiol. 116:67 (1982); and, Tushinski and Stanley, J. Cell. Physiol. 122:221 (1985). In the assay 50 $\mu$l of $\alpha$MEM were pipetted into each well of a 96-well flat bottom microtiter plate. 50 $\mu$l samples to be assayed for CSF-1 were dispensed into the first well of each row. 50 $\mu$l volumes were transferred down each row with mixing (approximately 8 pipettings) in each well before transfer to the next well thereby producing a twofold dilution series. Thereafter, 50 $\mu$l of cell suspension (composed of $5 \times 10^3$ to $10 \times 10^3$ frozen or fresh non-adherent murine bone marrow cells) was added to each well. The cultures were then incubated t 37°C in a humidified atmosphere containing 10% $CO_2$ in air. After 5 to 7 hours, 25 $\mu$l of tritiated thymidine ([3]HTdr) (New England Nuclear, Boston, MA), (80 Ci/mM specific activity) containing $\alpha$MEM+ (80 $\mu$Ci/ml) was added to each well. The cultures were then incubated until a total of 24 hours had elapsed since placing the bone marrow cells into the wells. Only bone marrow cells cultured in the presence of biologically active CSF-1 incorporated [3]HTdr in a dose-dependent manner. Bone marrow cells cultured in the absence of active CSF-1 incorporated only background levels of [3]HTdr.

After 24 hours of culture, the assays were harvested by mashing. To this end, 12.5 $\mu$l of 5% NP40 detergent was dispensed into each well and then the culture placed on a vibrator for 1 minute. Vibration caused lysis of the cells but the nuclei were left intact. Thereafter, 12.5 $\mu$l of 1% solution of anti-foam A emulsion (Sigma Chemical Company, St. Louis, MO) was added to each well and the microtiter plate vibrated for approximately 10 seconds. Thereafter, the nuclei were collected on glass fiber filters in the mashing unit. The filters were then dried and their radioactivity determined by liquid scintillation counting.

Units of activity were calculated as the quantity of analog CSF-1 that induced 50% of maximal [3]HTdr incorporation. Thus, if a 50 $\mu$l sample generated one-half maximal [3]HTdr incorporation at a dilution of 1:5, 1 unit was said to be contained in one-fifth of 50 $\mu$l or 10 $\mu$l. The sample would therefore contain 1,000 divided by 10

or 100 U/μl of activity.

## EXAMPLE 1

### Synthesis of Mutant CSF-1 Gene

14 oligonucleotides, designated as CSF-A through CSF-N in FIGURE 3, were individually synthesized with an Applied Biosystems Model 380A DNA synthesizer using the manufacturer's instructions. As illustrated in FIGURES 2 and 3, the composition of the nucleotides were selected so as to form complementary ends with each other to facilitate the assembling of the oligonucleotides into larger duplexed DNA fragments. To this end, with the exception of the 5′ and 3′ ends of oligonucleotides CSF-A and CSF-B, respectively, and the 3′ and 5′ ends of oligonucleotides CSF-M and CSF-N, respectively, all of the oligonucleotides were constructed to cooperatively form cohesive terminals each being 8 bp in length.

As further illustrated in FIGURE 2, several of the nucleotides in the synthetic oligos were changed from the cDNA sequence set forth in FIGURE 1 thereby to provide 24 unique restriction enzyme cleavage sites starting from the 5′ terminal of the mutated CSF-1 gene and extending to the 3′ terminal of this gene. The following unique restriction enzyme cleavage sites were provided: Kpn I; Sca I; Bst X I; Pst I; Aha II; Aat II; Sal I; Acc I; Hinc II; Afl II; Hind III; Ban II; Bsp 1286; HgiA I; Nsp II; Sst I; Stu I; Ava I; Xho I; Ssp I; Asu II; Bsm I; BamH I; and, Xba I.

The oligonucleotides CSF-A and CSF-B were configured to include nucleotides directly 5′ to the mutant CSF-1 gene that encoded the following six amino acids : Val-Pro-Leu-Asp-Lys-Arg. These six amino acids from the carboxyl-terminal portion of the yeast pre-pro-α leader sequence for high level expression of CSF-1 in yeast hosts, discussed below in Example 3.

As illustrated in FIGURES 2 and 3, the oligonucleotides CSF-A through CSF-N when assembled in duplexed form generally correspond to the cDNA for the CSF-1 gene set forth in FIGURE 1, but with a significant portion of the 3′ section of the cDNA removed. In the particular embodiment of the present invention illustrated by this Example, the mutant CSF-1 gene terminated at nucleic acid No. 474 (amino acid residue No. 158) in FIGURE 1. This corresponds to nucleic acid No. 492 in FIGURE 2 (amino acid residue No. 164).

The 14 chemically synthesized oligonucleotides set forth in FIGURE 3 were assembled into two preliminary duplexed fragments, each 280 bp in length. The first preliminary fragment, Fragment I, was composed of oligonucleotides CSF-A through CSF-H and the second preliminary fragment, Fragment II, was composed of CSF-G through CSF-N. The fragments were assembled by standard enzymatic ligation. However, before carrying out the ligation, the 5′ ends of the oligonucleotides were phosphorylated so that the fragments could be assembled. The 5′ ends of oligonucleotides CSF-A and CSF-N were not, however, phosphorylated, thereby to prevent end-to-end or tail-to-tail ligation of Fragments I and II. Phosphorylation was carried out at 37°C for one hour in a reaction mixture containing the following: the 14 oligonucleotides in FIGURE 3 (10 μg/ml each); 1 mM dATP; T4 polynucleotide kinase (10 U/μg DNA); 70 mM Tris-HC1 (pH 7.6); 10 mM MgCl2; and, 5 mM dithiothreitol. After one hour, the reaction was terminated by heating at 60°C for 10 min.

Following completion of the phosphorylation procedure, the synthesized oligonucleotides were ligated together to form duplexed preliminary Fragments I and II. The ligations were carried out at room temperature for 5 min. in a reaction mixture containing the following components: oligonucleotides (3.3 μg/ml each); T4 DNA ligase (1 U/μg DNA); 50 mM Tris-HCl (pH 7.4); 10 mM MgCl2; 10 mM dithiothreitol; 1 mM spermidine; and, 1 mM dATP. After ligation, the DNA fragments resulting therefrom were resolved by electrophoresis on low-melt agarose gels (Sea-Plaque, FMC Corporation, Rockland, ME) containing 89 mM Tris-borate (pH 8.0) and 2 mM EDTA. The nucleic acid bands which migrated at the expected sizes of preliminary Fragments I and II were sliced from the gel and then purified by an Elutip-d ion exchange chromatography column (Scheicher and Schuell, Keeno, NH) according to the manufacturer's instructions.

The preliminary Fragments I and II were then digested with restriction enzymes to form the three final DNA fragments F-1, F-2 and F-3, each for subcloning. Fragment I was digested with the restriction enzymes Hind III and Sst I by standard procedures, for instance as detailed by Maniatis et al., supra, beginning at page 104. By this digestion procedure, Fragment I was divided into a first 175 bp Kpn I-Hind III DNA fragment, designated as F-1, and a second 95 bp Hind III-Sst I fragment, designated as F-2. Preliminary Fragment II was digested with Sst I to form a 230 bp Sst 1-Xba I fragment, designated as F-3.

The three final fragments F-1, F-2 and F-3 were subcloned using standard techniques. To this end, the F-1 and F-2 fragments were ligated to plasmid pUC 19 which had been pretreated with restriction enzymes Kpn I-Hind III or Hind III/Sst I, respectively, using standard methods, such as disclosed by Maniatis et al., supra, beginning at page 229. The pUC 19 plasmid is widely commercially available. The F-3 fragment was ligated to the plasmid pGem4 (Promega Biotec, Madison, WI) which had been predigested with the restriction enzymes Sst I and Xba I.

The recombinant plasmids were then transformed into E. coli strain MM 294 made competent by the procedures of Hanahan, J. Mol. Biol. 166:557 (1983). In the procedure colonies of MM 294 are picked off of a fresh streak from a frozen stock of the cells (one 2.5 mm diameter colony per 10-15 ml) and dispersed in 1 ml of SOB medium by moderate vortexing. SOB medium is composed of 2% (w/v) Bacto Tryptone, 0.5% (w/v) yeast extract, 10 mM NaCl, 2.5 mM KCl, 10 mM MgCl2, 10 mM MgSO4. The medium was autoclaved and stock Mg2+(1 M MgCl2.6H2O + 1 M MgSO4.7H2O sterile filtered) was used to make the medium 20 mM in Mg2+, after which the mixture was sterile filtered through a prerinsed 22 μm filter unit. The final pH of the mixture was 6.8 to 7.0. The cells dispersed in SOB were used to inoculate a prerinsed flask of SOB medium: 10-30 ml in a 300 ml flask.

The culture was then incubated at 37°C, 275 revs/min., until the cell density was $4 \times 10^7$ to $7 \times 10^7$/ml (absorbance at 550 nm = 0.45 to 0.55 for DH1: in about 2 to 2.5 hours). The cells were collected into 50 ml polypropylene tubes and placed on ice for 10 to 15 min. and then pelleted at 250 revs/ml for 12 min. and 4°C. The cells were resuspended in 1/3 vol transformation buffer (TFB) by gentle vortexing, placed on ice for 10-15 min. and then pelleted again at 2,5000 rev/min. for 10 min. at 4°C. TFB was composed of 10 mM K-MES (pH6.2), 100 mM RbCl, 45 mM $MnCl_2.4H_2O$, 10 mM $CaCl_2.2H_2O$, 3 mM $HCoCl_3$. One M MES was adjusted to pH 6.3 using KOH, sterile filtered, and stored at -20°C. All salts were added as solids. The solution was sterile filtered through a prerinsed 0.22 μm filter unit and stored at 4°C for over one year.

Next, the pellet was resuspended in TFB at 1/12.5 of the original volume of cells (2.5 ml of the culture was concentrated in 200 μl, one discrete transformation). Fresh DMSO was added to 3.5% (7 μl/200 ml), swirled, and left on ice for 5 min. Thereafter, dithiothreitol was added to 75 mM, swirled and left on ice for 10 min. Another equal portion of DMSO was added and the cells then incubated for 5 min. on ice. Samples (210 μl) were then placed into chilled 17 mm x 100 mm polypropylene tubes (Falcon No. 2059). The DNA was added (less than 10 μl) and the mixture swirled and incubated on ice for 30 min. The mixture was then heat pulsed without agitation at 47°C for 90 seconds, and then placed on ice for 1 to 2 min. Thereafter, 800 μl of SOC medium (approximately 20°C) was added and the tubes incubated at 37°C, 225 rev/min. for 1 hour. SOC medium was composed of SOB medium containing 20 mM-glucose.

An appropriate fraction of the culture was pipetted into a pool of SOB medium (100 to 200 μl) on a L-Broth plate, with appropriate antibiotics, and spread gently and minimally using a bent (L-shaped) Pasteur pipet. The plates were then incubated at 37°C to establish colonies. Transformants were selected using the phenotypic identifier present in the plasmids (ampicillin, 75 μg/ml). After transformed hosts for the three subclones were identified, the sequences of each of the subclones was confirmed by the DNA sequencing procedure set forth above in Example A. The transformed hosts containing the desired DNA fragments F-1, F-2 and F-3 were sequenced by standard procedures. Thereafter, the DNA was removed and purified therefrom, as discussed above.

EXAMPLE 2

Expression of Functional Analog CSF-1 From Synthesized DNA Clones

To confirm that the synthesized mutant CSF-1 gene set forth in FIGURE 2 encoded a functional CSF-1 protein, the three subcloned DNA fragments F-1, F-2 and F-3 were ligated into plasmid pα3 to form a recombinant shuttle vector, designated as pαFCSF-1, to direct high level expression of analog CSF-1 in yeast host cells. The starting plasmid pα3 is on deposit with the American Type Culture Collection (ATCC), 12301 Parklawn Drive, Rockville, MD 20852, U.S.A. under Accession No. 53220. As shown in FIGURE 4, the pα3 plasmid includes an origin of replication and an Amp$^r$ resistant gene from plasmid pBR 332 (thick line portion). The pα3 plasmid also includes the 2 μ circle origin of replication and the Trp base I gene for selection of transformed yeast host (Trp-auxotrophs), thin line portion in FIGURE 4. The CSF-1 sequences, open box portions in FIGURE 4, were fused to the downstream (3') end of the α factor sequences. The pα3 plasmid also includes a linking oligonucleotide (shown in solid box portion) having cohesive Kpn I and Xba I compatible Spe I sites, for convenient ligation to the synthetic CSF-1 fragments (Fragments F-1, F-2 and F-3) prepared above in Example 1.

The pα3 vector was digested with the restriction enzymes Kpn I and Spe I. The enzyme Spe I generates a cohesive terminal that is compatible with the Xba I-generated cohesive terminal. to form the pαFCSF-1 shuttle vector, a four-way ligation was performed with: the Kpn I-Spe I digested vector: the F-1 (Kpn I-Hind III) 175 bp synthetic DNA fragment; the F-2 (Hind III-Sst I) 95 bp synthetic DNA fragment; and, the F-3 (Sst I-Xba I) 230 bp synthetic DNA fragment, using the standard techniques, such as set forth in Maniatis et al., supra. In the ligation procedure, approximately 50 nanograms ("ng") of the vector fragment was ligated together with approximately 10 ng each of the three synthetic DNA fragments. Next, the ligation mixture was transformed into E. coli strain RR1 using standard transformation techniques, for instance, as set forth in Bolivar et al., Gene 2:95 (1977); and, Peacock et al., Biochem. Biophys. Acta. 65:243 (1981). This strain of E. coli is widely commercially available. The host cells were grown in culture, removed from the culture and then lysed. Plasmids from the host cells that were transformed were checked for correct orientation of the mutant CSF-1 gene fragments within the plasmid by standard restriction enzyme analysis, for instance using the techniques discussed by Maniatis et al., supra at 374, and by Smith and Birnstiel, Nucl. Acid. Res. 3:2387 (1976).

After confirming that the three DNA fragments were ligated in proper relative location, the recombinant DNA shuttle vector, pαFCSF-1, was then transformed into yeast strain 79 (α, Trp 1-1, Leu 2-1) of S. cerevisiae for selection of Trp+ transformants by standard techniques. Prior to transformation, the strain 79 was grown in culture in YEPD medium (1% [wt/vol] yeast extract, 2% [wt/vol] peptone, 2% [wt/vol] glucose), to a density of $2 \times 10^7$ cells/ml. Cells were harvested by centrifugation at 1000 x g for 5 minutes at 22°C, and then the resulting pellet was washed with sterile, distilled water.

The yeast cells were then concentrated by resuspending in 1/10 vol. of SED (1 M sorbitol, 25 mM EDTA [pH 8.0], and 50 mM dithiothreitol) and incubating for 10 minutes at 30°C. The cell-buffer mixture was then centrifuged for 5 minutes at 300 x g. The pellet was washed once with 1/10 vol. of 1 M sorbitol and the cells resuspended in 1/10 volume of SCE (1 M sorbitol, 0.1 M sodium citrate [pH 5.8], 0.01 M EDTA). Glusulase, to break down the cell walls, in an amount of $10^{-3}$ vol., was added to the solution and then the solution incubated at 30°C for 30 minutes with occasional gentle shaking. The presence of spheroplasts was assayed by dilution 10 μl of the yeast cells into a drop of 5% sodium dodecyl sulfate (SDS) (wt/vol) on a microscope slide to observe for "ghosts" at 400 x phase contrast. The cell mixture was then centrifuged at 300 x g for 3 minutes. The resulting pellet was twice washed with 1/10 vol. of 1 M sorbitol. The pellet was then washed once with CaS (1 M sorbitol, 10 mM $CaCl_2$).

The yeast spheroplasts were then transformed with the previously prepared expression vector in a procedure adapted from Beggs, supra. The pelleted spheroplasts were suspended in 1/200 vol. of CaS and then divided into 100 μl aliquots in 1.5 ml Eppendorf tubes. Then, from 1 to 10 μl of the plasmid DNA were added to each aliquot (0.5 to 5 μg). The mixture was incubated at room temperature for 15 minutes and then 1 ml of polyethylene glycol (PEG) (20% PEG 4000, 10 mM $CaCl_2$, 10 mM Tris HCl [pH 7.4]) was added to each aliquot to promote DNA uptake. After 15 minutes at room temperature, the mixture was centrifuged for 5 minutes at 350 x g. The resulting pellet was resuspended in 150 μl of SOS (10 ml of 2 M sorbitol, 6.7 ml of YEPD medium, 0.13 ml of 1 M $CaCl_2$, 27 μl of 1% tryptophan and 3.7 ml of water). This mixture was incubated for 20 minutes at 30°C. The cells were then plated.

Prior to plating the protoplast/DNA mixture, selective plates were preincubated at 37°C. 3 ml of melted top agar (45°C), composed of: 18.2 ml of sorbitol; 2 gm agar; 0.6 gm Difco yeast nitrogen base (without amino acids); 2 gm glucose; 0.1 ml of 1% adenine; 0.4 ml of 1% uracil; and, amino acids as required, was then added to each aliquot of transformed cells and the tube contents poured on the selective plates. The plates were incubated from 2-4 days at 30°C. Colonies which developed in the Trp minus medium contained plasmids that have the Trp 1 gene, i.e., those that are transformed.

Prior to biological assay, the transformants were grown in 20-50 ml of YEPD at 30°C to stationary phase. At the time of harvest, the protease inhibitors phenyl methyl sulfonyl flouride (PMSF) and Pepstatin A were added to final concentrations of 1 mM and 10 μM, respectively. The cells were then removed by centrifugation at 400 x g and the medium was filtered through a 0.45 micron cellulose acetate filter (Corning Glass Works, Corning, New York). The sterile supernatants were stored at 4°C. The resulting supernatants, as assayed with the colony forming assay and the thymidine proliferation assay, contained approximately 45,000 CSF-1 CFU/ml or 1 μg of recombinant CSF-1 per ml of yeast fermentation fluid.

The discovery by applicants that the mutant CSF-1 gene of the present invention encodes functional protein leads to the conclusion that the active site of the CSF-1 molecule is contained within the amino acid sequence of CSF-1 set forth in FIGURE 2. It also confirms that, for reasons not entirely understood, the portion of the CSF-1 cDNA located 3' of nucleic acid No. 474 could be responsible for inhibition of expression of full length, biologically active CSF-1. Through this discovery, biologically active CSF-1 can now be produced in commercial quantities, for instance through large scale yeast fermentation. Heretofore, this has not been possible.

## EXAMPLE 3

### In Vitro Mutagenesis of CSF-1 cDNA

As noted above, the nucleic acid sequence of the cDNA for human CSF-1 is set forth in FIGURE 1. The cDNA for human CSF-1 is prepared by the method of Kawasaki et al., supra, by use of the Okayama-Berg expression vector as detailed in Mol. Cell. Biol. 2:161 (1982). A mutated gene for CSF-1 is prepared by in vitro mutagenesis of the CSF-1 cDNA. This is carried out by digesting plasmid DNA containing the CSF-1 cDNA with the restriction enzymes Sca I and Bam HI to produce a linear cDNA fragment extending from nucleic acid No. 17 to nucleic acid No. 469 in FIGURE 1. Thereafter, the resulting DNA fragment is treated with T4 DNA polymerase to remove the 5' overhang at the Bam HI terminal. Nco I linkers are added to the 3' end of the isolated cDNA by standard procedures, for instance as set forth in Maniatis et al., supra, to enable the cDNA fragment to be ligated to the Nco I site on the pα3 shuttle vector. The Nco I linkers, of the composition: GGGTAACCATGGCCC, include the stop codon TAA. The Nco I linkers are digested with Nco I restriction enzymes to generate a cohesive 3' end. The resulting Sca I-Nco I cDNA fragment is purified by electrophoresis through agarose gel.

An oligonucleotide is prepared to add back the nucleotides which were removed from the 5' terminal of the cDNA fragment by digestion of the plasmid cDNA with the restriction enzyme Sca I. The oligonucleotide also includes a promoter sequence for high level CSF-1 expression in a yeast system and a 5' cohesive terminal for linking the oligonucleotide to a cloning vector. The composition of the oligonucleotide, as shown in TABLE 1 below, includes a Kpn I cohesive 5' terminal followed by the nucleic acids encoding the carboxyl-terminal portion of the yeast pre-pro α leader sequence and then amino acids: Glu-Glu-Val-Ser-Glu-Tyr.

## TABLE 1

5'CT TTG GAT AAA AGA GAG GAG GTG TCG GAG T    3'

3' CAT GGA AAC CTA TTT TCT CTC CTC CAC AGC CTC A    5'

The resulting mutant CSF-1 gene extends from nucleic acid No. 1 to nucleic acid No. 469 in FIGURE 1. The analog CSF-1 encoded by this gene is expressed in yeast host cells by use of the pα3 shuttle vector described above in Example 1. To form a shuttle vector, designated as pαFmutCSF-1, a 3-way ligation is performed with the Kpn I-Nco I digested pα3 plasmid, the Kpn I-Sca I linking oligonucleotide (Table 1), and the Sca I-Nco I CSF-1 cDNA fragment. The resulting shuttle vector is transformed into E. coli strain MM294 using the procedure detailed in Example 1. The transformed E. coli hosts are selected and the plasmid DNA contained therein analyzed by restriction enzyme analysis to confirm that the oligonucleotide, the mutant CSF-1 gene and the pα3 plasmid are ligated in proper relative location. Thereafter, the shuttle vector is transformed into yeast strain 79 using the procedure set forth in Example 2. As in Example 2, the yeast supernatants are assayed with the colony forming assay and the thymidine proliferation assay detailed in Examples B and C.

## Claims

1. A biologically active recombinant mutant analog polypeptide or protein corresponding in amino acid composition to a native polypeptide or protein, but differing from the native polypeptide or protein by deletion or substitution of hydrophobic amino acids characteristic of a transmembrane region.

2. An analog polypeptide or protein according to claim 1, wherein the native polypeptide or protein is a lymphokine or lymphokine receptor.

3. An analog polypeptide or protein according to claim 2, wherein the native polypeptide or protein is human CSF-1.

4. An analog polypeptide or protein comprising an amino acid sequence corresponding to amino acids 1-166 of Figure 1.

5. An anlog polypeptide or protein comprising an amino acid sequence corresponding to amino acids 1-158 of Figure 1.

6. A mutant DNA sequence encoding an analog polypeptide or protein according to any one of claims 1 to 5.

7. A mutant DNA sequence having a nucleotide sequence corresponding to the nucleotide sequence of Figure 2.

8. A plasmid vector for expression of recombinant protein or polypeptide in a prokaryotic or eukaryotic unicellular organism, comprising a DNA sequence according to any of claims 6 to 7.

9. A plasmid vector for expression of recombinant protein or polypeptide yeast, comprising a DNA sequence according to any of claims 6 to 7.

10. A process for expressing a biologically active recombinant mutant analog polypeptide or protein coresponding in amino acid composition to a native polypeptide or protein, but differing from the native polypeptide or protein by deletion or substitution of hydrophobic amino acids characteristic of a transmembrance region, comprising culturing a host organism comprising a plasmid vector according to any of claims 6 to 7 and recovering the expressed polypeptide or protein from fermentation media.

CLAIMS FOR THE FOLLOWING CONTRACTING STATES: AT, ES

1. A process for preparing a biologically active recombinant mutant analog polypeptide or protein corresponding in amino acid composition to a native polypeptide or protein, but differing from the native polypeptide or protein by deletion or substitution of hydrophobic amino acids characteristic of a transmembrane region, comprising assembling by the formation of peptide bonds successive amino acid residues to provide the analog polypeptide or protein.

2. A process for preparing a biologically active recombinant mutant analog polypeptide or protein corresponding in amino acid composition to a native polypeptide or protein, but differing from the native polypeptide or protein by deletion or substitution of hyrdophobic amino acids characteristic of a transmembrane region, comprising assembling a mutant DNA sequence encoding the analog polypeptide or protein in a plasmid expression vector, transforming a suitable prokaryotic or eukaryotic unicellular host organism with the vector, culturing the host organism, and recovering expressed polypeptide or protein from fermentation media.

3. A process as claimed in claim 2, wherein the native polypeptide or protein is a lymphokine or lymphokine receptor.

4. A process as claimed in claim 3, wherein the native polypeptide or protein is human CSF-1.

5. A process as claimed in claim 4, wherein the analog polypeptide or protein comprises an amino acid sequence corresponding to amino acids 1-166 of Figure 1.

6. A process as claimed in claim 4, wherein the analog polypeptide or protein comprises an amino acid sequence corresponding to amino acids 1-158 of Figure 1.

7. A process as claimed in claim 4, wherein the mutant DNA sequence encoding the analog polypeptide or protein comprises a nucleotide sequence corresponding to the nucleotide sequence of Figure 2.

8. A process for the preparation of nucleic acid coding for a biologically active recombinant mutant analog polypeptide or protein corresponding in amino acid composition to a native polypeptide or protein, but differing from the native polypeptide or protein by deletion or substitution of hydrophobic amino acids characteristic of a transmembrane region, the process comprising coupling together successive nucleotides.

9. A process as claimed in claim 8, wherein the native polypeptide is a lymphokine or a lymphokine receptor.

10. A process as claimed in claim 9, wherein the nucleotide sequence corresponds to the nucleotide sequence of Figure 2.

CLAIMS FOR THE FOLLOWING CONTRACTING STATE: GR

1. A process for preparing a biologically active recombinant mutant analog polypeptide or protein corresponding to amino acid composition to a native polypeptide or protein, but differing from the native polypeptide or protein by deletion or substitution of hydrophobic amino acids characteristic of a transmembrane region, comprising assembling by the formation of peptide bonds successive amino acid residues to provide the analog polypeptide or protein.

2. A process for preparing a biologically active recombinant mutant analog polypeptide or protein corresponding in amino acid composition to a native polypeptide or protein, but differing from the native polypeptide or protein by deletion or substitution of hyrdophobic amino acids characteristic of a transmembrane region, comprising assembling a mutant DNA sequence encoding the analog polypeptide or protein in a plasmid expression vector, transforming a suitable prokaryotic or eukaryotic unicellular host organism with the vector, culturing the host organism, and recovering expressed polypeptide or protein from fermentation media.

3. A process as claimed in claim 2, wherein the native polypeptide or protein is a lymphokine or lymphokine receptor.

4. A process as claimed in claim 3, wherein the native polypeptide or protein is human CSF-1.

5. A process as claimed in claim 4, wherein the analog polypeptide or protein comprises an amino acid sequence corresponding to amino acids 1-166 of Figure 1.

6. A process as claimed in claim 4, wherein the analog polypeptide or protein comprises an amino acid sequence corresponding to amino acids 1-158 of Figure 1.

7. A process as claimed in claim 4, wherein the mutant DNA sequence encloding the analog polypeptide or protein comprises a nucleotide sequence corresponding to the nucleotide sequence of Figure 2.

8. A DNA sequence encoding an analog polypeptide or protein according to any one of claims 1-6.

9. A mutant DNA sequence having a nucleotide sequence corresponding to the nucleotide sequence of Figure 2.

10. A plasmid vector for expression of recombinant polypeptide or protein in a prokaryotic or eukaryotic unicellular organism, comprising a DNA sequence according to any of claims 8-9.

```
GAG GAG GTG TCG GAG TAC TGT AGC CAC ATG ATT GGG AGT GGA CAC CTG CAG TCT CTG CAG    60
Glu Glu Val Ser Glu Tyr Cys Ser His Met Ile Gly Ser Gly His Leu Gln Ser Leu Gln    20

CGG CTG ATT GAC AGT CAG ATG GAG ACC TCG TGC CAA ATT ACA TTT GAG TTT GTA GAC CAG    120
Arg Leu Ile Asp Ser Gln Met Glu Thr Ser Cys Gln Ile Thr Phe Glu Phe Val Asp Gln    40

GAA CAG TTG AAA GAT CCA GTG TGC TAC CTT AAG AAG GCA TTT CTC CTG GTA CAA TAC ATA    180
Glu Gln Leu Lys Asp Pro Val Cys Tyr Leu Lys Lys Ala Phe Leu Leu Val Gln Tyr Ile    60

ATG GAG GAC ACC ATG CGC TTC AGA GAT AAC ACC CCC AAT GCC ATC GCC ATT GTG CAG CTG    240
Met Glu Asp Thr Met Arg Phe Arg Asp Asn Thr Pro Asn Ala Ile Ala Ile Val Gln Leu    80

CAG GAA CTC TCT TTG AGG CTG AAG AGC TGC TTC ACC AAG GAT TAT GAA GAG CAT GAC AAG    300
Gln Glu Leu Ser Leu Arg Leu Lys Ser Cys Phe Thr Lys Asp Tyr Glu Glu His Asp Lys    100

GCC TGC GTC CGA ACT TTC TAT GAG ACA CCT CTC CAG TTG CTG GAG AAG GTC AAG AAT GTC    360
Ala Cys Val Arg Thr Phe Tyr Glu Thr Pro Leu Gln Leu Leu Glu Lys Val Lys Asn Val    120

TTT AAT GAA ACA AAG AAT CTC CTT GAC AAG GAC TGG AAT ATT TTC AGC AAG AAC TGC AAC    420
Phe Asn Glu Thr Lys Asn Leu Leu Asp Lys Asp Trp Asn Ile Phe Ser Lys Asn Cys Asn    140

AAC AGC TTT GCT GAA TGC TCC AGC CAA GGC CAT GAG AGG CAG TCC GAG GGA TCC TCC AGC    480
Asn Ser Phe Ala Glu Cys Ser Ser Gln Gly His Glu Arg Gln Ser Glu Gly Ser Ser Ser    160

CCG CAG CTC CAG GAG TCT GTC TTC CAC CTG CTG GTG CCC AGT GTC ATC CTG GTC TTG CTG    540
Pro Gln Leu Gln Glu Ser Val Phe His Leu Leu Val Pro Ser Val Ile Leu Val Leu Leu    180

GCC GTC GGA GGC CTC TTG TTC TAC AGG TGG AGG CGG CGG AGC CAT CAA GAG CCT CAG AGA    600
Ala Val Gly Gly Leu Leu Phe Tyr Arg Trp Arg Arg Arg Ser His Gln Glu Pro Gln Arg    200

GCG GAT TCT CCC TTG GAG CAA CCA GAG GGC AGC CCC CTG ACT CAG GAT GAC AGA CAG GTG    660
Ala Asp Ser Pro Leu Glu Gln Pro Glu Gly Ser Pro Leu Thr Gln Asp Asp Arg Gln Val    220

GAA CTG CCA GTG                                                                     672
Glu Leu Pro Val                                                                     224
```

*Fig. 1.*

Kpn I        ←— CSF-A —→     Sca I     Bst X I        Pst I

```
      ctttggataaaagaGAGGAGGTGTCGGAGTACTGTAGCCACATGATTGGGAGTGGACACCTtCAGTCTCTGCAGCGGCTGATTGACAGT  93
      catggaaacctattttctcTCCTCCACAGCCTCATGACATCGGTGTACTAACCCTCACCTGTGGAaGTCAGAGACGTCGCCGACTAACTGTCA
      ValProLeuAspLysArgGluGluValSerGluTyrCysSerHisMetIleGlySerGlyHisLeuGlnSerLeuGlnArgLeuIleAspSer  31
```

←—— CSF-B ——→


Aha II                  Sal I                 Afl II
Aat II             Acc I                Hind III
    ←—— CSF-C ——→     Hinc II              ←—— CSF-E ——→

```
      CAGATGGAGACgTCGTGCCAAATTACATTTGAGTTTGTcGACCAAGAACAGTTGAAAGATCCAGTGTGCTACCTTAAGAAaGCtTTTCTCCTG  186
      GTCTACCTCTGcAGCACGGTTTAATGTAAACTCAAACAgCTGGTTCTTGTCAACTTTCTAGGTCACACGATGGAATTCTTtCGaAAAGAGGAC
      GlnMetGluThrSerCysGlnIleThrPheGluPheValAspGlnGluGlnLeuLysAspProValCysTyrLeuLysLysAlaPheLeuLeu  62
```

←———CSF-D ——→                                         ←—— CSF-F —→


                                                          Sst I
                                                          Ban II
                                                           Bsp 1286
                                                           HgiA I
            ←—— CSF-G ——→                        Nsp II

```
      GTACAATACATAATGGAGGACACCATGCGCTTCAGAGATAACACCCCCAATGCAATCGCCATTGTGCAGCTtCAGGAgCTCTCTTTGAGGCTG  279
      CATGTTATGTATTACCTCCTGTGGTACGCGAAGTCTCTATTGTGGGGGTTACGGTAGCGGTAACACGTCGAaGTCCTcGAGAGAAACTCCGAC
      ValGlyTryIleMetGluAspThrMetArgPheArgAspAsnThrProAsnAlaIleAlaIleValGlnLeuGlnGluLeuSerLeuArgLeu  93
```

←—— CSF-H ——→

*Fig. 2A.*

024947?

```
                                   Stu I                                                    Xho I
                                    |          <──── CSF-I ────>                             |
                                    |                                                       | Ava I
                                    |                                                       |  |
AAGA GCTGCTTCACtAAGGATTATGAAGAGCATGACAAGG CCTGCGTCCGAACTTTCTATGAGACACCTCTCCAGT TGGTC GAGAAGGTCAAG  > 372
TTCT CGACGAAGTGaTTCCTAATACTTCTCGTACTGTTCC GGACGCAGGCTTGAAAGATACTCTGTGGAGAGGTCAACGA GCTC TTCCAGTTC
LysSerCysPheThrLysAspTyrGluGluHisAspLysAlaCysValArgThrPheTyrGluThrProLeuGlnLeuLeuGluLysValLys  124
                                        <──── CSF-J ────>
```

```
  <──── CSF-K ────>                                Ssp I                                              Bsm I
                                                    |     Asu II        <──── CSF-M ────>              |
                                                    |      |                                          |
AATGTCTTTAATGAAACAAAGAATCTCCTTGACAAGGACTGGAATA TTTTt tcgA AGAACTGCAACAACAGCTTTGCTGAATGCTCCAGCCAg  > 464
TTACAGAAATTACTTTGTTTCTTAGAGGAACTGTTCCTGACCTTATAAAA aagc TTCTTGACG TTGTTGTCGAAACGACTTACGAGGTCGGTc
AsnValPheAsnGluThrLysAsnLeuLeuAspLysAspTrpAsnIlePheSerLysAsnCysAsnAsnSerPheAlaGluCysSerSerGln  155
  <──── CSF-L ────>                                              <──── CSF-N ────>
```

```
                BamH I
                 |   Xba I
                 |    |
GGCCATGAGAGGCAGTCCGAGGGATCCTaat       499
CCGGTACTCTCCGTCAGGCTCCCTAGGAttagatc   499
GlyHisGluArgGlnSerGluGlySerEnd        164
```

*Fig. 2B.*

CSFA: ctttggataaaagaGAGGAGGTGTCGGAGTACTGTAGCCACATGATTGGGAGTGGACACCTtC

CSFB: CAGAGACTGaAGGTGTCCACTCCCAATCATGTGGCTACAGTACTCCGACACCTCCTCtctttatccaaaggtac

CSFC: AGTCTCTGCAGCGGCTGATTGACAGTCAGATGGAGACgTCGTGCCAAATTACATTTGAGTTTGTcGACCAAG

CSFD: CAACTGTTCTTGGTCgACAAACTCAAATGTAATTTGGCACGAcGTCTCCATCTGACTGTCAATCAGCCGCTG

CSFE: AACAGTTGAAAGATCCAGTGTGCTACCTTAAGAAaGCtTTTCTCCTGGTACAATACATAATGGAGGACACCA

CSFF: GAACGCATGGTGTCCTCCATTATGTATTGTACCAGGAGAAaGCtTTCTTAAGGTAGCACACTGGATCTTT

CSFG: TGCGCTTCAGAGATAACACCCCCAATGCCATCGCCATTGTGCAGCTtCAGGAgCTCTCTTTGAGGCTGA

CSFH: AGAGAAGCAGCTCTTCAGCCTCAAAGAGAGcTCCTGaAGCTGCACAATGGCGATGGCATTGGGGGTGTTATCTCT

CSFI: GCTGCTTCACtAAGGATTATGAAGAGCATGACAAGGCCTGCGTCCGAACTTTCTATGAGACACCTCTCCAGT

CSFJ: CTCgAGCAACTGGAGAGGTGTCTCATAGAAAGTTCGGACGCAGGCCTTGTCATGCTCTTCATAATCCTTaGT

CSFK: TGCTcGAGAAGGTCAAGAATGTCTTTAATGAAACAAAGAATCTCCTTGACAAGGACTGGAATATTTTttcgA

CSFL: GCAGTTCTTcgaaAAAATATTCCAGTCCTTGTCAAGGAGATTCTTTGTTTCATTAAAGACATTCTTGACCTT

CSFM: AGAACTGCAACAACAGCTTTGCTGAATGCTCCAGCCAgGGCCATGAGAGGCAGTCCGAGGGATCCTaat

CSFN: ctagattAGGATCCCTCGGACTGCCTCTCATGGCCcTGGCTGGAGCATTCAGCAAAGCTGTTGTT

*Fig.3.*

Fig. A.

SYNTHETIC CSF-1 OLIGONUCLEOTIDES

0249477

Fig. 5.